# EUROPEAN PATENT SPECIFICATION

(11) **EP 3 504 177 B1**
(45) Date of publication and mention of the grant of the patent: **21.07.2021**
(21) Application number: 17801509.5
(22) Date of filing: 25.08.2017
(51) Int. Cl.: C07C 45/00, C07C 45/30, C07C 49/11

(54) **PROCESS FOR THE PREPARATION OF CYCLIC KETONES**
VERFAHREN ZUR HERSTELLUNG VON CYCLISCHEN KETONEN
PROCÉDÉ DE PRÉPARATION DE CÉTONES CYCLIQUES

(30) Priority: 26.08.2016 IN 201611029181; 01.09.2016 IN 201611029978
(43) Date of publication of application: 03.07.2019
(73) Proprietor: SRF LIMITED, Gurgaon 122003 (IN)
(72) Inventor: GUPTA, Gopesh, Gurgaon Haryana 122003 (IN); SONI, Chandresh, Gurgaon Haryana 122003 (IN); KUMAR, Anil, Haryana Gurgaon 122003 (IN); GUPTA, Hanuman Prasad, Gurgaon Haryana 122003 (IN); KUMAR, Kapil, Gurgaon Haryana 122003 (IN); ANAND, Rajdeep, Gurgaon Haryana 122003 (IN)
(74) Representative: AWA Sweden AB
(86) International application number: PCT/IN2017/050358
(87) International publication number: WO 2018/037428

(56) References cited:
- EP-A1- 0 731 075
- EP-A1- 0 889 019
- EP-A1- 2 607 344
- US-A- 3 898 246

## Description

### Field of the invention

The present invention provides a process for the preparation of cyclic ketones.

### Background of the invention

The catalytic hydrogenation of phenols to cyclic ketones is widely known. The EP Publication No. 0,889,019 A1 and EP Publication No. 0,890,565 A1 describe methods for preparing cyclohexanones by hydrogenation of the corresponding phenols in the presence of alkanes or water as solvent. The EP Patent No. 2,607,345 describes a process for preparing cyclohexanones by hydrogenation of the corresponding phenols in the presence of alcohol as solvent.

### Summary of the invention

In one aspect, the present invention provides a process for the preparation of a compound of Formula II, comprising;
a) contacting a compound of Formula I with hydrogen in the presence of hydrogenation catalyst to obtain the compound of Formula II, and 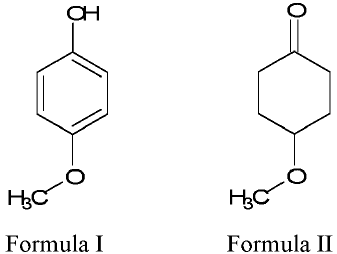
b) isolating the compound of Formula II obtained from step a), wherein the step a) takes place in the absence of a solvent and wherein the hydrogenation catalyst of step a) is palladium supported on carbon and containing an alkali metal component selected from alkali metal hydroxides, preferably sodium hydroxide and potassium hydroxide or alkali metal carbonate, preferably sodium carbonate and potassium carbonate.

Disclosed herein is a process for the preparation of a compound of Formula II, comprising;
a) contacting a compound of Formula I with hydrogen in the presence of hydrogenation catalyst to obtain the compound of Formula II, and 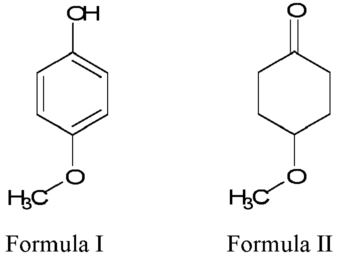
b) isolating the compound of Formula II obtained from step a), wherein the step a) takes place in the absence of a solvent.

Further disclosed herein is a process for the preparation of a compound of formula II, comprising;
c) contacting a mixture of the compound of formula II and a compound of formula III with hypohalous acid to obtain the compound of formula II, and 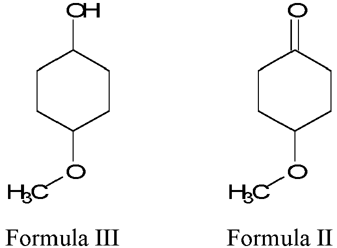
d) isolating the compound of formula II obtained from step c).

Yet further disclosed herein is a process for the preparation of a compound of formula II, comprising;
e) contacting a compound of formula III with hypohalous acid to obtain the compound of formula II, and 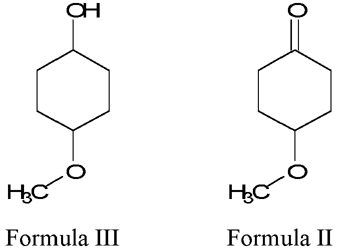
f) isolating the compound of formula II obtained from step e).

### Detailed description of the invention

The compound of Formula I, which is used as a starting material in step a) may be obtained commercially.

The hydrogenation catalyst used in the step a) may be 1 to 30% by weight of the palladium supported on a carrier. The alkali metal-containing palladium catalysts are preferred. The carrier of the palladium catalyst or an alkali metal-containing palladium catalyst is preferably a carbon or alumina. The alkali metal component may be selected from the group consisting of alkali metal hydroxides e.g., sodium hydroxide and potassium hydroxide or alkali metal carbonate e.g., sodium carbonate, and potassium carbonate.

The preferred alkali metal components are selected from the group consisting of sodium hydroxide and sodium carbonate.

The hydrogenation reaction of step a) can be performed by using 0.1 to 5.0 mole equivalents of the alkali metal component with respect to the palladium catalyst and 0.00005 to 0.1 mole equivalents of palladium catalyst with respect to the starting material i.e., compound of formula I.

The hydrogenation reaction can be carried out at a pressure selected in the range from atmospheric to 20 bar and at a temperature selected in the range from 60°C to 150°C. The catalyst used in the step a) can be recycled back after isolation from reaction mass by filtration.

The compound of Formula II is isolated by any method known in the art, for example, chemical separation, acid-base neutralization, distillation, evaporation, column chromatography and filtration or mixture thereof.

The hypohalous acid used in the step c) and step e) is itself prepared by contacting the trichloroisocyanuric acid with water or by contacting the metal hypochlorite with an organic or inorganic acid or mixture thereof wherein the metal hypochlorite is selected from the group consisting of calcium hypochlorite, sodium hypochlorite and potassium hypochlorite or mixture thereof and the organic acid is selected from the group consisting of formic acid, acetic acid, chloroacetic acid, dichloroacetic acid, trichloroacetic acid, chlorodifluoroacetic acid, difluoroacetic acid and trifluoroacetic acid or mixture thereof. The inorganic acid is selected from group consisting of hydrochloric acid, nitric acid, sulfuric acid, phosphoric acid, hydrofluoric acid and hydrobromic acid or mixture thereof.

The reaction of step c) and step e) is carried out in acidic medium at a pH below 7 and at a temperature selected in the range of -20°C to 100°C in the presence or absence of a solvent, wherein the solvent is selected from the group consisting of water, alcohols, ketones, acetonitrile, tetrahydrofuran and dioxane or mixtures thereof.

The ketone is selected from the group consisting of methyl isobutyl ketone, cycloheaxnone, methyl t-butylketone or mixture thereof.

The reaction can be carried out in the acidic medium which can be achieved either by addition of the acid to the mixture of compound of Formula II and Formula III followed by the addition of metal hypohalide or both acid and metal hypohalide can be added simultaneously to the mixture of compound of Formula II and Formula III maintaining the acidic reaction mass.

While the present invention has been described in terms of its specific embodiments, certain modifications and equivalents will be apparent to those skilled in the art and are intended to be included within the scope of the present invention.

The following examples are given by way of illustration and therefore should not be construed to limit the scope of the present invention.

### PREPARATION OF 4-METHOXYCYCLOHEXANONE

### Example 1, comparative example:

The mixture of 4-methoxycyclohaxanol and 4-methoxycyclohexanone (100 g) was taken in a round bottomed flask and aqueous sodium hypochlorite (6%w/w, 930gm) was added with continuous stirring at 20°C over the period of 3 hours to 4 hours. The acidic medium was maintained in the reaction mixture with simultaneous addition of concentrated hydrochloric acid. The progress of reaction was monitored by gas chromatography. After completion of the reaction, the excess of hypochlorite was neutralized using aqueous sodium sulfite solution and the crude product thus obtained was distilled to get the title compound.
Yield (%): 90

### Example 2:

The 4-methoxyphenol (100 g) and palladium-carbon catalyst (1.0g, 5% Pd containing sodium carbonate) were taken together in 400 mL hydrogenation reactor. The hydrogenation was conducted at 130°C and the pressure was maintained at 2 to 4 Bar. The progress of the reaction was monitored with gas chromatography. The reaction mass was cooled and filtered to isolate the catalyst. The 11% sodium hypochlorite (62g) and acetic acid (3g) was added. The crude product thus obtained was distilled to get the title compound.
Yield(%): 95%

## Claims

1. A process for the preparation of a compound of formula II, comprising;
a) contacting a compound of Formula I with hydrogen in the presence of hydrogenation catalyst to obtain the compound of Formula II, and 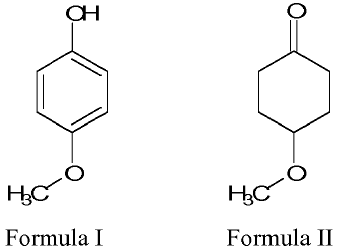
b) isolating the compound of Formula II obtained from step a),
wherein the step a) takes place in absence of a solvent and wherein the hydrogenation catalyst of step a) is palladium supported on carbon and containing an alkali metal component selected from alkali metal hydroxides, preferably sodium hydroxide and potassium hydroxide or alkali metal carbonate, preferably sodium carbonate and potassium carbonate.

2. The process as claimed in claim 1, wherein the hydrogenation reaction is carried out at a pressure selected in the range from atmospheric to 20 bar and at a temperature selected in the range from 60°C to 150°C.

## Patentansprüche

1. Verfahren zur Herstellung einer Verbindung der Formel II, das Folgendes umfasst:
a) Inkontaktbringen einer Verbindung der Formel I mit Wasserstoff in Gegenwart von Hydrierkatalysator zum Erhalt der Verbindung der Formel II und 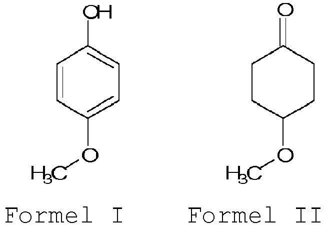
b) Isolieren der aus Schritt a) erhaltenen Verbindung der Formel II,
wobei Schritt a) in Abwesenheit von Lösungsmitteln erfolgt und der Hydrierkatalysator von Schritt a) auf Kohle geträgertes Palladium ist und eine aus Alkalimetallhydroxiden, vorzugsweise Natriumhydroxid und Kaliumhydroxid, oder Alkalimetallcarbonat, vorzugsweise Natriumcarbonat und Kaliumcarbonat, ausgewählte Alkalimetallkomponente enthält.

2. Verfahren nach Anspruch 1, wobei die Hydrierungsreaktion bei einem Druck im Bereich von Normaldruck bis 20 bar und bei einer Temperatur im Bereich von 60 °C bis 150 °C durchgeführt wird.

## Revendications

1. Procédé pour la préparation d'un composé de formule II, comprenant :
a) la mise en contact d'un composé de Formule I avec de l'hydrogène en la présence d'un catalyseur d'hydrogénation pour obtenir le composé de Formule II, et 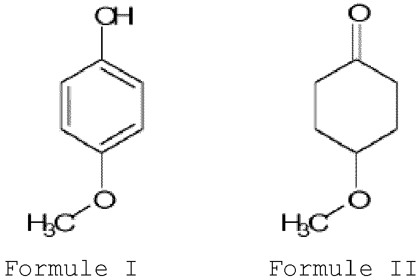
b) l'isolement du composé de Formule II obtenu de l'étape a),
l'étape a) ayant lieu en l'absence d'un solvant et le catalyseur d'hydrogénation de l'étape a) étant du palladium supporté sur carbone et contenant un composant de métal alcalin choisi parmi des hydroxydes de métal alcalin, préférablement l'hydroxyde de sodium et l'hydroxyde de potassium, et un carbonate de métal alcalin, préférablement le carbonate de sodium et le carbonate de potassium.

2. Procédé selon la revendication 1, la réaction d'hydrogénation étant mise en œuvre à une pression choisie dans la plage de la pression atmosphérique à 20 bars et à une température choisie dans la plage de 60 °C à 150 °C.
